# EUROPEAN PATENT SPECIFICATION

(11) **EP 2 566 463 B1**
(45) Date of publication and mention of the grant of the patent: **25.03.2015**
(21) Application number: 10760801.0
(22) Date of filing: 01.09.2010
(51) Int. Cl.: A61K 9/20, A61K 31/704

(54) **EXTENDED RELEASE THIOCOLCHICOSIDE TABLETS**
THIOCOLCHICOSIDTABLETTEN MIT VERLÄNGERTER FREISETZUNG
COMPRIMES DE THIOCOLCHICOSIDE A LIBERATION PROLONGEE

(30) Priority: 12.03.2010 TR 201001903
(43) Date of publication of application: 13.03.2013
(73) Proprietor: Ali Raif Ilac Sanayi Ve Ticaret A.S., Istanbul (TR)
(72) Inventor: BULGUR , Abdullah, Haseyad II. Kisim No:228 Istanbul (TR)
(74) Representative: Bulut, Pinar
(86) International application number: PCT/TR2010/000174
(87) International publication number: WO 2011/112161

(56) References cited:
- EP-A1- 2 074 990
- WO-A1-99/39717

## Description

### Technical Field:

Present invention is related to extended release pharmaceutical composition containing 8 to 16 mg thiocolchicoside with once a day dosage regime. The composition contains a polymer which controls the release of thiocolchicoside. 35 to 65% of total thiocolchicoside is released in the first hour in 0.1 N hydrochloric acid medium. The composition developed is equivalent to the conventional tablets taken twice daily.

### Previous Technique:

Thiocolchicoside is the synthesized derivative of colchicoside, a natural glycoside, by adding sulphur (Formula I). Its chemical name is *N*-[3-(β-D-glucopyranosyloxy)-1,2-dimethoxy-10-(methylthio)-9-oxo-5,6,7,9-tetrahydrobenzo[*a*]heptalen-7-yl]acetamide and its molecular weight is 563.618.

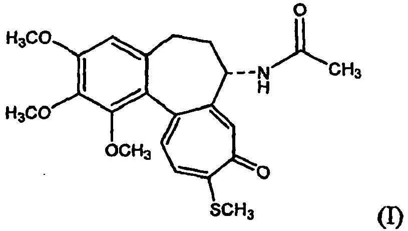

Thiocolchicoside, which has the pharmacological muscle relaxing effect, reduces the contractions from central nervous system or completely relieves them similar to the situation of spastic hypertonus. Thiocolchicoside prevents residual spasm by reducing the passive resistance.

Muscle relaxing effect of thiocolchicoside is also observed in visceral muscles. Thiocolchicoside exerts its effect through central nervous system and it does not cause motor plaque paralysis.

Thiocolchicoside is a specific gamma-aminobutiric acid (GABA) receptor antagonist. It has also glycinergic agonist effect. As a result, thiocolchicoside does not block voluntary muscle movements and does not constitute any risk of respiration paralysis.

In the commercially available, conventional, immediate release forms, the active metabolite of thiocolchicoside, 3-Desmethylthiocolchicine reaches the highest plasma concentration approximately in one hour. Its muscle relaxing effect starts in 1-2 hours and the effect increases cumulatively with the increasing doses. Elimination half of life of the drug in plasma is 4-5 hours. Most of the dose taken is renally excreted and the remaining part is excreted through extrarenal ways.

WO1999/03971 explains the nasal application of thiocolchicoside.

WO 1998/036751 is about the application of thiocolchicoside on oral mucosa.

WO 1996/041635 is about the concomitant administration of thiocolchicoside with diclofenac salts.

EP2074990 is related to the controlled release flurbiprofen and muscle relaxant combinations.

### Advantages of the invention:

As the elimination half life of the active metabolite Desmethylthiocolchicine, reaching maximum plasma concentration in one hour, is 4-5 hours, very low plasma concentrations are observed at the time of the next dose. As a result, deep releases occur in plasma concentrations with conventional tablets of 4-8 mg, taken every 12 hours. These releases get deeper with repeated doses. Present invention makes the plasma concentration more stable by reducing the deepness of these releases thanks to extended release. It is also more convenient for the patient with single daily dose.

### Description of the invention:

Present invention is related to the formulation of extended release tablets containing 8 - 16 mg thiocolchicoside, which are bioequivalent to the commercially available, conventional 4 and 8 mg tablets administered twice daily.

The developed extended release tablet makes 35 to 65% immediate release in order to provide the sufficiently effective blood level and reach the Cmax level of the conventional tablets. Then, It makes extended release the remaining thiocolchicoside in 5 hours or more in order to keep the plasma concentration efficient for longer durations.

The tablet developed consists of the separate parts, one of which provides immediate release and the other provides extended release. 55-56% of thiocolchicoside content of the tablet is in the extended release part and the remaining 35/45% of it is in immediate release part. These two separate parts may be compressed as two layered tablets. In another form of the invention, extended release part constitutes the core of the tablet. Immediate release part is coated on the core tablet.

Extended release part is the matrix type formulation. Cellulose derivative polymers are used in matrix type of formulation. Preferred cellulose derivative used as polymer is hydroxypropyl methylcellulose and more preferably, hydroxypropyl methylcellulose 4000 cps commercially known as Methocel K4M CR.

In coated tablet formulation, appropriate coating polymers may be used in the immediate release part. Preferred polymer is hydroxypropyl methylcellulose, more preferably hydroxypropyl methylcellulose 3 cps, commercially known as Methocel E3-LV is used. Examples related to the invention are given below and the invention is not limited to these examples given.

### Example 1:

Formulations of the extended release and immediate release parts are given in Table 1.1 and 1.2.

**Table 1.1 Immediate release part**

| | | |
|---|---|---|
| 1 | Thiocolchicoside | 8.0mg |
| 2 | Lactose monohydrate | 161.2mg |
| 3 | Pregelatinized Starch | 40.0mg |
| 4 | Sucrose | 5.0mg |
| 5 | Gelatin | 2.5mg |
| 6 | Talc | 8.0mg |
| 7 | Magnesium Stereate | 1.3mg |
| | TOTAL | 226.0mg |

Preparation: 1,2,3 are sieved and mixed. 4 and 5 are dissolved by heating in water. Powder mix is granulated with this solution. Granules are dried at 60°C. It is sieved from 30 mesh. 6 and 7 are sieved and added.

These are mixed for 5 minutes.

**Table 1.2 Extended release part**

| | | |
|---|---|---|
| 1 | Thiocolchicoside | 8.0mg |
| 2 | Lactose monohydrate | 133.5mg |
| 3 | Povidone K-30 | 6.2mg |
| 4 | Hydroxypropyl methylcellulose | 50.0mg |
| 5 | Colloidal silicone dioxide | 0.3mg |
| 6 | Magnesium Stereate | 2.0mg |
| | TOTAL | 200.0mg |

Preparation: 1 and 2 are sieved and mixed. It is dissolved in 3 water/alcohol (1:1) mix. Powder mix is granulated with this solution. Granules are dried in 55°C. 4 and 5 are added and sieved from 30 mesh. 6 is sieved from 30 mesh and added and mixed for 5 minutes. Two layers are compressed in double feeding tablet machine in minimum 10 kp hardness. Tablets were tested for one hour in 500 ml 0.1 N HCl medium at 100 rpm in basket type dissolution device and then for 10 hours in 500 ml pH 6.8 phosphate buffer.

**Table 1,3 Dissolution profile**

| 0.1N HCl | | | | pH 6.8 Phosphate buffer | | | | | | | | |
|---|---|---|---|---|---|---|---|---|---|---|---|---|
| Time* | 15m | 30m | 60m | 1hr | 2 hr | 3hr | 4hr | 5 hr | 6 hr | 7 hr | 8 hr | 9hr |
| Dissolution, % | 48 | 53 | 61 | 68 | 74 | 80 | 85 | 88 | 92 | 96 | 99 | 100 |

| | | | | | | | | | | | | |
|---|---|---|---|---|---|---|---|---|---|---|---|---|
| * m=minutes, hr=hour | | | | | | | | | | | | |

### Example 2:

Tablets with parts of immediate and extended release are obtained as in Example I and tested by the methods given in Example I.

**Table 2.1 Immediate release part**

| | | |
|---|---|---|
| 1 | Thiocolchicoside | 6.5mg |
| 2 | Lactose monohydrate | 162.7mg |
| 3 | Pregelatinized starch | 40.0mg |
| 4 | Sucrose | 5.0mg |
| 5 | Gelatin | 2.5mg |
| 6 | Talc | 8.0mg |
| 7 | Magnesium Stereate | 1.3mg |
| | TOTAL | 226.0mg |

**Table 2,2 Extended release part**

| | | |
|---|---|---|
| 1 | Thiocolchicoside | 9.5mg |
| 2 | Lactose monohydrate | 132.0mg |
| 3 | Povidone K-30 | 6.2mg |
| 4 | Hydroxypropyl methylcellulose | 50.0mg |
| 5 | Colloidal silicone dioxide | 0.3mg |
| 6 | Magnesium Stereate | 2.0mg |
| | TOTAL | 200.0mg |

**Table 2,3 Dissolution profile**

| 0.1N HCl | | | | pH 6.8 Phosphate buffer | | | | | | | | |
|---|---|---|---|---|---|---|---|---|---|---|---|---|
| Time * | 15m | 30m | 60m | 1h | 2 hr | 3hr | 4hr | 5 hr | 6 hr | 7 hr | 8 hr | 9hr |
| Dissolution, % | 40 | 44 | 53 | 59 | 65 | 71 | 77 | 82 | 86 | 90 | 94 | 98 |

| | | | | | | | | | | | | |
|---|---|---|---|---|---|---|---|---|---|---|---|---|
| * m=minutes, hr=hour | | | | | | | | | | | | |

### Example 3:

Tablets with parts of immediate and extended release are obtained as in Example I and tested by the methods given in Example I.

**Table 3,1 Immediate release part**

| | | |
|---|---|---|
| 1 | Thiocolchicoside | 6.5mg |
| 2 | Lactose monohydrate | 162.7mg |
| 3 | Pregelatinized starch | 40.0mg |
| 4 | Sucrose | 5.0mg |
| 5 | Gelatin | 2.5mg |
| 6 | Talc | 8.0mg |
| 7 | Magnesium stereate | 1.3mg |
| | TOTAL | 226.0mg |

**Table 3,2 Extended release part**

| | | |
|---|---|---|
| 1 | Thiocolchicoside | 9.5mg |
| 2 | Lactose monohydrate | 123.0mg |
| 3 | Povidone K-30 | 6.2mg |
| 4 | Hydroxypropyl methylcellulose | 59.0mg |
| 5 | Colloidal silicone dioxide | 0.3mg |
| 6 | Magnesium stereate | 2.0mg |
| | TOTAL | 200.0mg |

**Table 3,3 Dissolution profile**

| 0.1N HCl | | | | pH 6.8 Phosphate buffer | | | | | | | | |
|---|---|---|---|---|---|---|---|---|---|---|---|---|
| Time* | 15m | 30m | 60m | 1h | 2 hr | 3hr | 4hr | 5 hr | 6 hr | 7 hr | 9hr | 12hr |
| Dissolution, % | 41 | 45 | 51 | 59 | 65 | 71 | 75 | 79 | 83 | 87 | 93 | 100 |

| | | | | | | | | | | | | |
|---|---|---|---|---|---|---|---|---|---|---|---|---|
| * m=minutes, hr=hours | | | | | | | | | | | | |

### Example 4:

Tablet core which provides extended release was coated with the immediate release part which contains the active ingredient.

**Table 4.1 Tablet core and the formulation of coating**

| Extended release part | | |
|---|---|---|
| 1 | Thiocolchicoside | 9.5mg |
| 2 | Lactose monohydrate | 122.9mg |
| 3 | Povidone K-30 | 6.2mg |
| 4 | Hydroxypropyl methylcellulose | 59.0mg |
| 5 | Colloidal silicone dioxide | 0.4mg |
| 6 | Magnesium stereate | 2.0mg |
| | TOTAL | 200.0mg |

| Coating with active ingredient | | |
|---|---|---|
| 7 | Thiocolchicoside | 6.5mg |
| 8 | hydroxypropyl methylcellulose | 3.4mg |
| 9 | Povidone K-30 | 0.5mg |
| 10 | Polyethylene glycole | 0.1mg |
| | TOTAL | 211.5mg |

Preparation: 1 and 2 are sieved and mixed. It is dissolved in 3 water/alcohol (1:1) mix. Powder mix is granulated with this solution. Granules are dried in 55°C. 4 and 5 are added and sieved from 30 mesh. 6 is sieved from 30 mesh and added and mixed for 5 minutes. 200 mg tablets are compressed in round, convex moulds.

7, 8, 9, 10 are dissolved in water and the coating solution is prepared. Tablets are coated until they weigh 211.5 mg at inlet air temperature of 50°C.

Tablets were tested for one hour in 500 ml 0.1 N HCl medium at 100 rpm and then for 12 hours in 500 ml pH 6.8 phosphate buffer. The dissolution profile obtained is shown in Table 4.2.

**Table 4,2 Dissolution test**

| 0.1N HCl | | | | pH 6.8 Phosphate buffer | | | | | | | | |
|---|---|---|---|---|---|---|---|---|---|---|---|---|
| Time* | 15m | 30m | 60m | 1hr | 2 hr | 3hr | 4hr | 5 hr | 6 hr | 7 hr | 9hr | 12hr |
| Dissolution, % | 40 | 45 | 51 | 58 | 65 | 70 | 75 | 79 | 83 | 87 | 90 | 98 |

| | | | | | | | | | | | | |
|---|---|---|---|---|---|---|---|---|---|---|---|---|
| * m=minutes, hr=hours | | | | | | | | | | | | |

### Example 5:

Tablets are obtained as in Example 4 and tested by the methods given in Example 4.

**Table 5.1 Tablet core and the formulation of coating**

| Extended release part | | |
|---|---|---|
| 1 | Thiocolchicoside | 4.5mg |
| 2 | Lactose monohydrate | 127.9mg |
| 3 | Povidone K-30 | 6.2mg |
| 4 | Hydroxypropyl methylcellulose | 59.0mg |
| 5 | Colloidal silicone dioxide | 0.4mg |
| 6 | Magnesium Stereate | 2.0mg |
| | TOTAL | 200.0mg |

| Coating with active ingredient | | |
|---|---|---|
| 7 | Thiocolchicoside | 3.5mg |
| 8 | Hidroksipropilmetil Selüloz | 3.4mg |
| 9 | Povidone K-30 | 0.5mg |
| 10 | Polyethylene glycole | 0.1mg |
| | TOTAL | 207.5mg |

**Tablo 5.2 Dissolution test**

| 0.1N HCl | | | | pH 6.8 Phosphate buffer | | | | | | | | |
|---|---|---|---|---|---|---|---|---|---|---|---|---|
| Time * | 15m | 30m | 60m | 1hr | 2 hr | 3hr | 4hr | 5 hr | 6 hr | 7 hr | 9hr | 12hr |
| Dissolution, % | 43 | 48 | 54 | 60 | 66 | 71 | 75 | 78 | 82 | 85 | 89 | 95 |

| | | | | | | | | | | | | |
|---|---|---|---|---|---|---|---|---|---|---|---|---|
| * m=minutes, hr=hours | | | | | | | | | | | | |

### Bioequivalence study

In this study, extended release thiocolchicoside tablet (Formula 4) was compared to commercially available, conventional immediate release, 2 Coltramyl 4mg tablets.

The study was performed as an open label pilot bioequivalence study on 12 volunteers. One group was administered 1 dose of Thiocolchicoside 16 mg SR (Formula 4) and the other group was given 2 Coltramyl 4mg tablets in 0 and 12th hours. 7 days of wash out period was used in the study.

Blood samples were taken 1 hour before Thiocolchicoside 16mg SR administration and 0.25, 0.50, 0.75, 1.00, 1.25, 1.50, 2.00, 4.00, 8.00, 10.00, 12.00, 16.00 and 24.00 hours after the administration in order to establish the thiocolchicoside serum concentration.

As the reference product 2 Coltramyl 4mg tablets were given twice daily, in order to determine the peak plasma levels after the second dose, plasma was re-tested in additional time points. As a result, 17 time points were determined. These are 1 hour before the administration and 0.25, 0.50, 0.75, 1.00, 1.25, 1.50, 2.00, 4.00, 12.00, 12.25, 12.50, 12.75, 13.00, 14.00, 16.00 and 24.00 hours after the administration.

5 ml of blood was taken from the volunteers each time. Mean AUC₀₋ₜ, AUC_{0-∞}, Cₘₐₓ and Cₘᵢₙ values were calculated from the serum measurements of all volunteers. Results are given in Table 6.1-6.3.

**Table 6,1 Results of the bioequivalence study**

| ADMINISTRATION | AUC₀₋ₜ | AUC_{0-∞} | Cₘₐₓ | Tₘₐₓ | Cₘᵢₙ |
|---|---|---|---|---|---|
| 2X2XColtramyl 4mg (0 and 12 hr) | 102.75 | 113.62 | 1st peak 18.98 | 0.75 hr | 0.32 |
| | | | 2nd peak 19,54 | 12.75 hr | 0.42 |
| Formulation 4 | 106.56 | 108.75 | 22.48 | 1 hr | 0.29 |

**Table 6.2: Findings of bioequivalence test - Thiocolchicoside 16 mg SR**

| Time (hr) | Plasma concentration ng/mL | | Parameter | Value | Unit |
|---|---|---|---|---|---|
| 0,00 | | | Intersection | 4,000994988 | ng/ml |
| 0,25 | 0,78 | | Gradient | 0,132397146 | h-1 |
| 0,5 | 10,63 | | t1/2 | 5,234251819 | h |
| 0,75 | 17,65 | | AUC 0-tn | 106,565 | h*ng/ml |
| 1 | 22,48 | | AUC 0-inf | 108,7553795 | h*ng/ml |
| 1,25 | 22,24 | | MRT | 4,433815091 | h |
| 1,5 | 22,02 | | Cmax | 22,48 | ng/ml |
| 2 | 21,65 | | Tₘₐₓ | 1 | h |
| 4 | 12,23 | | tn | 24 | h |
| 8 | 2,56 | | Ctn | 0,29 | ng/ml |
| 10 | 1,25 | | 1,25 | 1,25 | 1,25 |
| 12 | 0,67 | | | | |
| 16 | 0,46 | | | | |
| 24 | 0,29 | | | | |

**Table 6,3: Findings of the bioequivalence test - 2XColtramyl 4 mg X 2 dose (0 and 12 hours)**

| Time (hr) | Plasma concentration ng/ml | | Parameter | Value | Unit |
|---|---|---|---|---|---|
| 0,00 | | | Intersection | 3,574079708 | ng/ml |
| 0,25 | 2,47 | | Gradient | 0,038619896 | h-1 |
| 0,5 | 15 | | t1/2 | 17,94411868 | h |
| 0,75 | 18,98 | | AUC 0-tn | 102,75 | h*ng/ml |
| 1 | 17 | | AUC 0-inf | 113,6252234 | h*ng/ml |
| 1,5 | 11,78 | | MRT | 12,74709984 | s |
| 2 | 8,08 | | Cmax | 19,54 | ng/ml |
| 4 | 2,77 | | Tₘₐₓ | 12,75 | s |
| 12 | 0,32 | | tn | 24 | s |
| 12,25 | 2,75 | | Ctn | 0,42 | ng/ml |
| 12,50 | 15,83 | | | | |
| 12,75 | 19,54 | | | | |
| 13 | 18,43 | | | | |
| 14 | 8,24 | | | | |
| 16 | 2,93 | | | | |
| 24 | 0,42 | | | | |

### Description of the figures

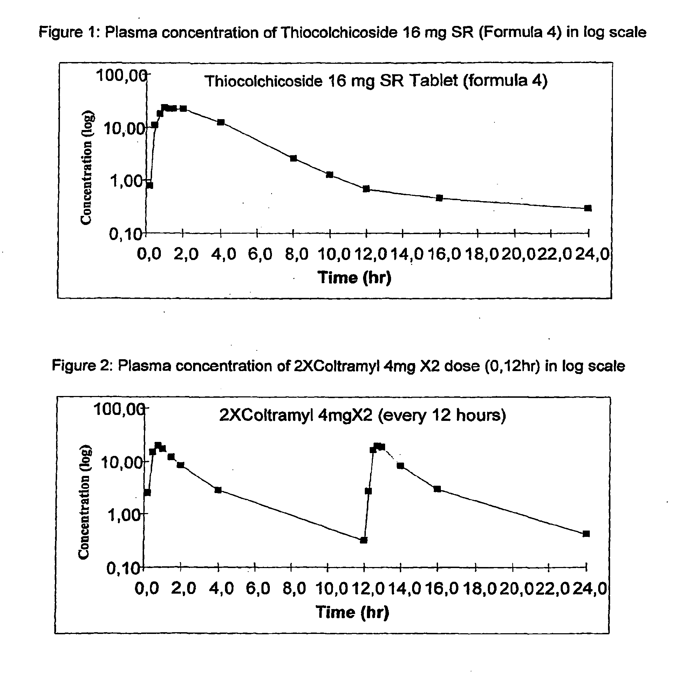

Figure 1: Plasma concentration of Thiocolchicoside 16 mg SR(Formula 4) in log scale
Figure 2: Plasma concentration of 2XColtramyl 4mg X2 dose (0,12hr) in log scale

## Claims

1. An extended release tablet composition comprising thiocolchicoside **characterized in that** the tablet composition comprises an immediate release part and an extended release part containing hydroxypropyl methylcellulose as gel forming polymer wherein immediate release part and extended release part comprise thiocolchicoside.

2. An extended release tablet according to claim 1 wherein the ratio of hydroxypropyl methylcellulose in the extended release part is 25-30% of extended release part in weight.

3. An extended release tablet according to the claim 1-2 wherein the viscosity of hydroxypropyl methylcellulose is 4000 cps (4 Pa.s).

4. An extended release tablet according to the claim 1 wherein each tablet contains 6 or 16 mg of thiocolchicoside.

5. An extended release tablet according to the claim 1 wherein extended release part comprises 55-65% and immediate release part comprises 35-45% of the total amount of thiocolchicoside in the tablet.

6. An extended release tablet according to claim 1 wherein tablet core comprises extended release part and coating on the core comprises immediate release part.

7. An extended release tablet according to claim 6; wherein
a) a core comprises 4.5 to 9.5mg thiocolchicoside, lactose monohydrate, polyvinylpyrrolidone, hydroxypropyl methylcellulose, colloidal silicone dioxide and magnesium stereate,
b) a coating on the core comprises 3.5 to 6.5mg thiocolchicoside, hydroxypropyl methylcellulose, polyvinylpyrrolidone and polyethylene glycol.

8. An extended release tablet according to claim 1 wherein the tablet is two layered tablet.

9. An extended release tablet according to claim 8; wherein
a) the extended release part comprises 8 to 9.5 mg of thiocolchicoside, lactose monohydrate, polyvinylpyrrolidone, hydroxypropyl methylcellulose, colloidal silicone dioxide and magnesium stereate.
b) the immediate release part comprises 6.5 to 8mg thiocolchicoside, lactose monohydrate, pregelatinized starch, sucrose, gelatin, talc and magnesium stereate.

10. An extended release tablet according to the previous claims wherein 35-65% of the total amount of active substance is released at first hour in 500 ml of 0.1 N hydrochloric acid at 100 rpm in a rotating basket type dissolution test equipment.

## Patentansprüche

1. Thiocolchicosid umfassende Tablettenzusammensetzung mit verlängerter Freisetzung, **dadurch gekennzeichnet, dass** die Tablettenzusammensetzung einen Teil mit sofortiger Freisetzung und einen Teil mit verlängerter Freisetzung enthaltend Hydroxypropylmethylcellulose als gelbildendes Polymer umfasst, wobei der Teil mit sofortiger Freisetzung und der Teil mit verlängerter Freisetzung Thiocolchicosid umfassen.

2. Tablette mit verlängerter Freisetzung nach Anspruch 1, wobei das Verhältnis von Hydroxypropylmethylcellulose in den Teil mit verlängerter Freisetzung 25-30 Gew.-% des Teils mit verlängerter Freisetzung ausmacht.

3. Tablette mit verlängerter Freisetzung nach Anspruch 1 oder 2, wobei die Viskosität der Hydroxypropylmethylcellulose 4000 cps (4 Pa·s) beträgt.

4. Tablette mit verlängerter Freisetzung nach Anspruch 1, wobei jede Tablette 8 oder 16 mg Thiocolchicosid enthält.

5. Tablette mit verlängerter Freisetzung nach Anspruch 1, wobei der Teil mit verlängerter Freisetzung 55-65% und der Teil mit sofortiger Freisetzung 35-45% der Gesamtmenge an Thiocolchicosid in der Tablette umfasst.

6. Tablette mit verlängerter Freisetzung nach Anspruch 1, wobei der Tablettenkern den Teil mit verlängerter Freisetzung umfasst und die Beschichtung auf dem Kern den Teil mit sofortiger Freisetzung umfasst.

7. Tablette mit verlängerter Freisetzung nach Anspruch 6, wobei
a) ein Kern 4,5 bis 9,5 mg Thiocolchicosid, Lactose-monohydrat, Polyvinylpyrrolidon, Hydroxypropylmethylcellulose, kolloidales Siliciumdioxid und Magnesiumstearat umfasst,
b) eine Beschichtung auf dem Kern 3,5 bis 6,5 mg Thiocolchicosid, Hydroxypropylmethylcellulose, Polyvinylpyrrolidon und Polyethylenglykol umfasst.

8. Tablette mit verlängerter Freisetzung nach Anspruch 1, wobei es sich bei der Tablette um eine zweischichtige Tablette handelt.

9. Tablette mit verlängerter Freisetzung nach Anspruch 8, wobei
a) der Teil mit verlängerter Freisetzung 8 bis 9,5 mg Thiocolchicosid, Lactose-monohydrat, Polyvinylpyrrolidon, Hydroxypropylmethylcellulose, kolloidales Siliciumdioxid und Magnesiumstearat umfasst,
b) der Teil mit sofortiger Freisetzung 6,5 bis 8 mg Thiocolchicosid, Lactose-monohydrat, vorgelatinisierte Stärke, Saccharose, Gelatine, Talkum und Magnesiumstearat umfasst.

10. Tablette mit verlängerter Freisetzung nach einem der vorherigen Ansprüche, wobei bei 100 U/min in einem Auflösungstestgerät vom Drehkorbtyp 35-65% der Gesamtmenge an Wirkstoff innerhalb der ersten Stunde in 500 ml 0,1 N Salzsäure freigesetzt wird.

## Revendications

1. Composition de comprimé à libération prolongée comprenant du thiocolchicoside **caractérisée en ce que** la composition de comprimé comprend une partie à libération immédiate et une partie à libération prolongée contenant de l'hydroxypropylméthylcellulose en tant que polymère gélifiant dans laquelle la partie à libération immédiate et la partie à libération prolongée comprennent du thiocolchicoside.

2. Comprimé à libération prolongée selon la revendication 1 dans lequel le rapport de l'hydroxypropylméthylcellulose dans la partie à libération prolongée est de 25 à 30 % de la partie à libération prolongée en poids.

3. Comprimé à libération prolongée selon la revendication 1 à 2 dans lequel la viscosité de l'hydroxypropylméthylcellulose est de 4000 cps (4 Pa.s).

4. Comprimés à libération prolongée selon la revendication 1 dans lequel chaque comprimé contient de 8 ou 16 mg de thiocolchicoside.

5. Comprimés à libération prolongée selon la revendication 1 dans lequel la partie à libération prolongée comprend de 55 à 65 % et la partie à libération immédiate comprend de 35 à 45 % de la quantité totale de thiocolchicoside dans le comprimé.

6. Comprimé à libération prolongée selon la revendication 1 dans lequel le noyau de comprimé comprend une partie à libération prolongée et un enrobage sur le noyau comprend une partie à libération immédiate.

7. Comprimé à libération prolongée selon la revendication 6 dans lequel :
a) un noyau comprend de 4,5 à 9,5 mg de thiocolchicoside, de lactose monohydraté, de polyvinylpyrrolidone, d'hydroxypropylméthylcellulose, de dioxyde de silicium colloïdal et de stéarate de magnésium,
b) un enrobage sur le noyau comprend de 3,5 à 6,5 mg de thiocolchicoside, d'hydroxypropylméthylcellulose, de polyvinylpyrrolidone et de polyéthylène glycol.

8. Comprimé à libération prolongée selon la revendication 1 le comprimé étant un comprimé à deux couches.

9. Comprimés à libération prolongée selon la revendication 8 dans lequel :
a) la partie à libération prolongée comprend de 8 à 9,5 mg de thiocolchicoside, de lactose monohydraté, de polyvinylpyrrolidone, d'hydroxypropylméthylcellulose, de dioxyde de silicium colloïdal et de stéarate de magnésium,
b) la partie à libération immédiate comprend de 6,5 à 8 mg de thiocolchicoside, de lactose monohydraté, d'amidon prégélatinisé, de saccharose, de gélatine, de talc et de stéarate de magnésium.

10. Comprimé à libération prolongée selon les revendications précédentes dans lequel de 35 à 65 % de la quantité totale de substance active est libérée dans la première heure dans 500 ml d'acide chlorhydrique 0,1 N à 100 tours/min dans un équipement d'essai de dissolution de type à panier rotatif.
